(19) Europäisches Patentamt
European Patent Office
Office européen des brevets

(11) **EP 3 148 961 B1**

(12) **FASCICULE DE BREVET EUROPEEN**

(45) Date de publication et mention de la délivrance du brevet:
**22.08.2018 Bulletin 2018/34**

(21) Numéro de dépôt: **15724710.7**

(22) Date de dépôt: **28.05.2015**

(51) Int Cl.:
***C07C 67/52*** *(2006.01)*
***C07C 69/157*** *(2006.01)*
***C07C 227/42*** *(2006.01)*
***C07C 229/26*** *(2006.01)*
***C07C 67/28*** *(2006.01)*

(86) Numéro de dépôt international:
**PCT/EP2015/061864**

(87) Numéro de publication internationale:
**WO 2015/181304 (03.12.2015 Gazette 2015/48)**

(54) **PROCÉDÉ DE PRÉPARATION D'UN SEL D'ACIDE ACÉTYLSALICYLIQUE ET D'UN ACIDE AMINÉ BASIQUE**

VERFAHREN ZUR HERSTELLUNG EINES SALZES AUS ACETYLSALICYLSÄURE UND EINER BASISCHEN AMINOSÄURE

METHOD FOR PREPARING A SALT OF ACETYLSALICYLIC ACID AND A BASIC AMINO ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.05.2014 FR 1454830**

(43) Date de publication de la demande:
**05.04.2017 Bulletin 2017/14**

(73) Titulaire: **Unither Pharmaceuticals**
**80084 Amiens (FR)**

(72) Inventeur: **ARNAL, Thierry**
**F-21000 Dijon (FR)**

(74) Mandataire: **Ipside**
**6, Impasse Michel Labrousse**
**31100 Toulouse (FR)**

(56) Documents cités:
**WO-A1-2011/039432**
**CN-A- 102 503 845**
**FR-A1- 2 950 625**
**FR-A1- 2 973 370**
**FR-A1- 2 992 641**
**US-A1- 2002 091 108**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 148 961 B1

## Description

La présente invention concerne un procédé de préparation d'un sel d'acide acétylsalicylique et d'un acide aminé basique, en particulier de lysine. Ce procédé permet notamment d'obtenir, avec un haut degré de pureté, un tel sel présentant une stabilité dans le temps particulièrement élevée.

L'acide acétylsalicylique est utilisé depuis de nombreuses années dans le domaine thérapeutique, en particulier pour ses effets antalgique, anti-inflammatoire, antipyrétique, anti-rhumatismal et antiagrégant plaquettaire. Sa solubilité dans l'eau est cependant limitée, si bien qu'il ne peut être administré que sous des formes galéniques le contenant à l'état solide, et par voie orale.

Afin d'améliorer la solubilité de l'acide acétylsalicylique, en particulier dans l'eau, il a été proposé par l'art antérieur de le présenter sous forme d'un sel d'acide aminé basique. L'acétylsalicylate de DL-lysine constitue ainsi à l'heure actuelle le principe actif de plusieurs médicaments.

Un inconvénient majeur de l'acétylsalicylate de DL-lysine est sa faible stabilité, notamment en raison de son caractère hygroscopique, si bien que les formulations pharmaceutiques qui le contiennent présentent une durée de conservation limitée. Cette instabilité a été expliquée par une chaine de réactions conduisant, en présence d'humidité, à la formation d'un produit de dégradation particulier, l'acide salicylique, dont la présence dans la formulation pharmaceutique est indésirable.

Il a été proposé par l'art antérieur des procédés de fabrication d'acétylsalicylate de lysine visant à améliorer la stabilité de ce dernier.

Le document FR 2 992 641 décrit un tel procédé mettant en oeuvre le mélange d'une solution alcoolique d'acide acétylsalicylique et d'une solution aqueuse de monoacétate de lysine.

On peut notamment citer le document US 2002/0091108, qui décrit un procédé de fabrication d'acétylsalicylate de lysine comprenant le mélange rapide d'acide acétylsalicylique et de lysine, dans des conditions d'excès molaire de lysine, ce mélange étant suivi d'un refroidissement du milieu réactionnel à 0 °C concomitant à l'ajout d'un grand volume d'acétone, pour permettre la formation de cristaux par agitation du milieu réactionnel pendant plusieurs heures. Un tel procédé est cependant long à mettre en oeuvre, et il est par conséquent associé à des coûts de revient élevés. En outre, le degré de pureté de l'acétylsalicylate de lysine qu'il permet d'obtenir est insuffisant pour satisfaire aux spécifications de la pharmacopée pour une utilisation en tant que principe actif de médicament.

Il a autrement été proposé par l'art antérieur, notamment illustré par le document WO 2011/039432, le document FR 2 973 370 ou encore le document WO 2011/039432, de préparer de l'acétylsalicylate de DL-lysine par mélange d'acide acétylsalicylique et de DL-lysine, dans des conditions de défaut molaire en DL-lysine. Le milieu réactionnel est ensuite dilué dans un grand volume d'acétone, de sorte à provoquer la précipitation du sel désiré. Il a cependant été constaté par les présents inventeurs que la stabilité du sel d'acide acétylsalicylique et de lysine ainsi obtenu était insuffisante. Ce procédé ne permet en outre pas d'obtenir ce sel avec un degré de pureté satisfaisant.

La présente invention vise à remédier aux inconvénients des procédés décrits par l'art antérieur pour la préparation d'acétylsalicylate de DL-lysine, notamment à ceux exposés ci-avant, en proposant un procédé qui permette l'obtention d'un tel sel, et, plus généralement, d'un sel d'acide acétylsalicylique et d'un acide aminé basique, qui présente une bonne stabilité, et un degré de pureté élevé. Un objectif supplémentaire de l'invention est que ce procédé soit simple, rapide et peu coûteux de mise en oeuvre.

A l'origine de l'invention, il a été découvert par les présents inventeurs que, contrairement à ce qui est proposé par l'art antérieur, qui préconise l'introduction dans le milieu réactionnel de quantités de réactifs telles qu'un des deux réactifs, l'acide acétylsalicylique ou l'acide aminé basique, se trouve en excès par rapport à l'autre, de sorte à influer sur le pH du milieu réactionnel, l'introduction dans le réacteur de synthèse de quantités strictement équimolaires de ces deux réactifs permet, de manière surprenante, d'obtenir un sel de l'acide acétylsalicylique et de l'acide aminé basique de haut degré de pureté et très stable, ceci avec un rendement de réaction élevé.

En particulier, il a été découvert par les présents inventeurs que le maintien d'un ratio stoechiométrique acide acétylsalicylique/acide aminé basique constant à une valeur de 1:1 dans le réacteur de synthèse, permet d'éviter sensiblement entièrement la formation de produits de réaction secondaires, notamment d'acide salicylique, qui est l'impureté majoritaire susceptible d'être générée dans le milieu réactionnel. Un tel ratio stoechiométrique prévient en effet l'occurrence de toute réaction d'hydrolyse, acide ou basique, du sel d'acide acétylsalicylique et de l'acide aminé basique se formant dans le réacteur de synthèse.

Ainsi, il est proposé selon la présente invention un procédé de préparation d'un sel d'acide acétylsalicylique et d'un acide aminé basique, qui comprend le mélange, dans un réacteur, d'une solution d'acide acétylsalicylique et d'une solution de l'acide aminé basique, à une température inférieure ou égale à 30 °C à pression atmosphérique. Ce mélange est réalisé par introduction progressive dans le réacteur, simultanément, de la solution d'acide acétylsalicylique et de la solution de l'acide aminé basique, dans des conditions telles que durant toute la durée de cette introduction, à chaque instant les quantités d'acide acétylsalicylique et dudit acide aminé basique introduites dans le réacteur sont équimolaires.

Par équimolaire, on inclut les ratios stoechiométriques acide acétylsalicylique/acide aminé basique compris entre 0,99:1 et 1:0,99.

On entend dans la présente description, par acide aminé, aussi bien les $\alpha$-acides aminés naturels, c'est-à-dire de configuration L, que leurs énantiomères de configuration D, ou un mélange des formes L et D, ainsi que leurs homologues, isomères et dérivés, tels que des acides aminés comportant un groupement protecteur.

Un acide aminé basique est défini dans la présente description de manière classique en elle-même, comme un acide aminé à chaîne latérale chargée positivement à pH neutre.

L'acide aminé basique peut notamment être choisi parmi les acides aminés polaires ionisables, plus particulièrement les acides aminés dibasiques.

Préférentiellement, l'acide aminé basique est la lysine, notamment la L-lysine ou la DL-lysine, le cas échéant sous forme de monohydrate, ou ses homologues, isomères ou dérivés.

L'acide aminé peut autrement être choisi parmi l'arginine, l'histidine et l'ornithine, ou leurs homologues, isomères ou dérivés.

L'acide acétylsalicylique peut être introduit dans le réacteur de synthèse en solution dans un solvant miscible à l'eau, tel qu'un alcool, notamment le méthanol, l'éthanol ou l'isopropanol, un éther, tel que le tétrahydrofurane, ou une cétone, notamment l'acétone, ou dans un mélange de tels solvants. Préférentiellement, on met en oeuvre une solution d'acide acétylsalicylique dans l'acétone.

L'acide aminé basique est quant à lui de préférence introduit dans le réacteur en solution aqueuse, obtenue notamment par dissolution de l'acide aminé basique dans de l'eau distillée.

Les paramètres permettant de contrôler le nombre de moles de chacun des réactifs introduit dans le réacteur sont la concentration molaire de la solution initiale du réactif, ainsi que son débit d'introduction dans le réacteur. Selon la présente invention, les valeurs de chacun de ces paramètres sont choisies en fonction les unes des autres, de sorte à assurer qu'à chaque instant, il soit introduit dans le réacteur la même quantité molaire d'acide acétylsalicylique et d'acide aminé basique. Il est du ressort de l'homme du métier d'effectuer un tel choix conjoint de valeurs de ces paramètres.

Dans des modes de mise en oeuvre particuliers de l'invention, la solution d'acide acétylsalicylique comprend de 0,8 à 0,9 mol/l d'acide acétylsalicylique. De telles concentrations permettent notamment une solubilisation optimale de l'acide acétylsalicylique dans l'acétone, en particulier dans des conditions de température comprise entre 10 et 30 °C.

La solution de l'acide aminé basique peut quant à elle comprendre une concentration comprise entre 4,5 mol/l et 5,5 mol/l dudit acide aminé basique. Une telle plage de concentration permet notamment avantageusement, d'une part, de former une solution dont la viscosité est suffisamment faible pour pouvoir l'introduire facilement dans le réacteur, au moyen d'une pompe doseuse classique en elle-même ; et, d'autre part, lorsque la solution est une solution aqueuse, d'en limiter la teneur en eau à une valeur qui ne soit pas susceptible d'empêcher la cristallisation ultérieure, dans le milieu réactionnel, du sel d'acide acétylsalicylique et de l'acide aminé basique se formant lors de la réaction.

Dans des modes de mise en oeuvre particuliers du procédé selon l'invention, l'introduction progressive dans le réacteur de la solution d'acide acétylsalicylique et de la solution d'acide aminé basique est réalisée avec un débit d'introduction de la solution d'acide acétylsalicylique compris entre 10 et 50 l/h, et un débit d'introduction de la solution d'acide aminé basique compris entre 2 et 15 l/h.

Ces débits d'introduction peuvent aussi bien être constants que variables. Dans ce dernier cas, leurs profils de variations sont similaires.

Lorsque l'introduction des solutions de réactifs dans le réacteur est réalisée au moyen de pompes doseuses, les fréquences de ces pompes sont en particulier réglées de sorte à être sensiblement identiques l'une à l'autre, pour permettre à chaque instant l'introduction dans le réacteur de quantités de réactifs strictement équimolaires.

Lors de la phase d'introduction des solutions de réactifs dans le réacteur de synthèse, l'exothermie de la réaction se produisant dans ce dernier, à partir de l'acide acétylsalicylique et de l'acide aminé basique, est maîtrisée par un contrôle de la température du milieu réactionnel, assurant que la température de celui-ci ne dépasse pas, préférentiellement, une valeur de 20 °C à pression atmosphérique. De préférence, la température du milieu réactionnel contenu dans le réacteur est maintenue comprise entre -10 °C et 20 °C, à pression atmosphérique, pendant toute la durée de la phase d'introduction des solutions de réactifs dans le réacteur. Ce contrôle de la température du milieu réactionnel peut être réalisé par tout moyen classique en lui-même, notamment par mise en oeuvre d'un réacteur comportant une chemise de refroidissement, à l'intérieur de laquelle est entraîné en circulation un liquide réfrigérant de température adéquate.

La plage de températures préconisée par la présente invention, non seulement permet d'éviter la formation de produits secondaires indésirables qui serait susceptible d'être provoquée par une température trop élevée du milieu réactionnel, mais, en outre, elle favorise notamment et de manière tout à fait avantageuse une bonne cristallisation du sel d'acide acétylsalicylique et d'acide aminé basique se formant dans le réacteur. Ce sel sera désigné par commodité, dans la suite de la présente description, par l'expression « sel d'intérêt ».

Préférentiellement, une agitation faible, par exemple comprise entre environ 200 et 500 tr/min lorsque le réacteur

mis en oeuvre présente une capacité de trois litres, est maintenue dans le réacteur durant toute la phase d'introduction des solutions de réactifs.

Dans des modes de mise en oeuvre particulièrement préférés de l'invention, le procédé comprend une phase dite de mûrissement des cristaux, selon laquelle le milieu réactionnel, formé à l'issue de la phase d'introduction de la solution d'acide acétylsalicylique et de la solution de l'acide aminé basique dans le réacteur, est maintenu sous agitation à une température comprise entre -15 °C et 20 °C, de préférence d'environ -10 °C, pendant une durée comprise entre 30 et 90 minutes, de préférence pendant 1 heure. Le milieu réactionnel ne subit préférentiellement aucune modification préalablement à cette phase de maintien sous agitation. En particulier, il ne lui est ajouté au préalable aucun solvant, ou aucun autre composant visant notamment à favoriser la précipitation du sel d'acide acétylsalicylique et de l'acide aminé basique sous forme de cristaux dans le milieu réactionnel. Ainsi, selon l'invention, la phase de mûrissement des cristaux est effectuée directement sur le milieu réactionnel issu du mélange de la solution d'acide acétylsalicylique et de la solution d'acide aminé basique, sans aucune étape intermédiaire. Cette phase peut en outre avantageusement être mise en oeuvre dans le réacteur de synthèse lui-même.

En cela, le procédé selon l'invention se révèle bien plus avantageux que les procédés préconisés par l'art antérieur, qui comportent une phase de précipitation du sel d'acide acétylsalicylique et de l'acide aminé basique par transfert du milieu réactionnel dans un second réacteur, et mélange avec un grand volume d'acétone.

Au contraire, selon l'invention, la précipitation du sel d'acide acétylsalicylique et de l'acide aminé basique est réalisée *in situ,* sans dilution préalable du milieu réactionnel, y compris lorsque ce dernier comporte en tant que solvant un mélange d'un solvant organique, notamment l'acétone, et d'eau. De manière surprenante, il a été découvert par les présents inventeurs que dans de telles conditions, la précipitation du sel d'intérêt, qui est pourtant très soluble dans l'eau, s'effectue sans baisse de rendement, et sans impact sur la qualité du sel formé.

Le contrôle conforme à l'invention de la température du milieu réactionnel lors de la phase de mûrissement des cristaux permet notamment d'éviter une telle solubilisation du sel d'intérêt dans le mélange de solvants contenu dans le réacteur, et ainsi d'augmenter le rendement du procédé selon l'invention.

Dans des modes de mise en oeuvre particuliers de l'invention, à l'issue de la phase de mûrissement des cristaux, le solide contenu dans le milieu réactionnel est isolé. A cet effet, il peut être mis en oeuvre toute technique conventionnelle, par exemple la technique de centrifugation ou la technique de filtration.

Le cas échéant, le solide ainsi isolé peut être lavé, à une ou plusieurs reprises, au moyen d'un solvant organique, tel qu'un alcool, une cétone, ou un mélange de tels solvants, de sorte à le débarrasser des éventuelles impuretés, telles par exemple que le produit de dégradation principal du sel d'intérêt, l'acide salicylique.

Après la ou les étape(s) de lavage éventuelle(s), la phase solide est de nouveau séparée de la phase liquide, et peut être soumise à séchage.

Dans des variantes de l'invention, à l'issue de la phase de mûrissement des cristaux, le solide contenu dans le milieu réactionnel est soumis à une étape de recristallisation du sel d'acide acétylsalicylique et dudit acide aminé basique, le cas échéant après avoir été isolé du milieu réactionnel, puis le cas échéant lavé au moyen d'un solvant organique.

Une telle étape de recristallisation permet avantageusement d'obtenir une meilleure hétérogénéité de taille des particules du sel d'acide acétylsalicylique et de l'acide aminé basique obtenu.

Elle peut être réalisée par toute méthode connue de l'homme du métier.

Préférentiellement, elle est réalisée dans un mélange de solvants comprenant de l'eau et un moins un alcool, par exemple l'éthanol ou le 2-propanol.

A titre d'exemple, l'étape de recristallisation peut être réalisée par la succession des phases suivantes, le solide ayant préalablement été isolé du milieu réactionnel, par exemple par filtration :

- mise en suspension du solide dans l'alcool, par exemple dans deux volumes d'alcool,
- ajout d'eau, par exemple de deux volumes d'eau, dans le mélange obtenu, de sorte à solubiliser le produit,
- ajout d'une nouvelle quantité d'alcool, par exemple de quatre volumes d'alcool,
- première phase de refroidissement, par exemple jusqu'à une température d'environ 25 °C, et amorçage de la recristallisation,
- deuxième phase de refroidissement, par exemple jusqu'à une température d'environ 5 °C, pendant quelques minutes.

De telles conditions opératoires permettent avantageusement d'obtenir des cristaux de sel d'acide acétylsalicylique et de l'acide aminé basique de taille homogène, avec une surface spécifique relativement faible, de sorte que l'absorption éventuelle, à la surface des cristaux obtenus, de solvants résiduels préjudiciables à la stabilité du produit

lors de sa conservation, est avantageusement minimisée.

A l'issue de l'étape de recristallisation, le solide obtenu, constitué du sel d'acide acétylsalicylique et de l'acide aminé basique avec un haut degré de pureté, est séparé de la phase liquide, et peut être soumis à une étape de séchage.

Dans des modes de réalisation particulièrement préférés de l'invention, le procédé comprend de préférence une étape finale de séchage du sel d'acide acétylsalicylique et de l'acide aminé basique obtenu.

Selon la présente invention, le séchage est préférentiellement réalisé en au moins deux étapes successives, comprenant :

- une première étape de séchage sous agitation, de préférence sous faible agitation, à une première pression P1 comprise entre 200 et 300 mbar, de préférence d'environ 250 mbar, et à une première température T1 comprise entre 20 et 25 °C, pendant une durée comprise entre 90 et 250 minutes, de préférence d'environ 230 minutes comprenant une durée de 15 minutes de montée en température ;

- une deuxième étape de séchage sous agitation, de préférence sous faible agitation, à une deuxième pression P2 comprise entre 10 et 30 mbar, de préférence d'environ 20 mbar, et à une deuxième température T2 comprise entre 40 et 50 °C, de préférence d'environ 42 °C, pendant une durée comprise entre 90 et 250 minutes, de préférence d'environ 235 minutes comprenant une durée de 15 minutes de montée en température.

Un tel procédé en deux étapes permet avantageusement de former un sel d'acide acétylsalicylique et de l'acide aminé basique qui comporte une teneur en eau résiduelle très faible, inférieure à 0,5 %, si bien qu'il présente une très bonne stabilité dans le temps, conservé sous atmosphère inerte.

De telles conditions de séchage progressif, par paliers successifs de montée en température et de diminution en pression, permettent en effet de sécher le sel d'intérêt rapidement et efficacement, et de l'obtenir sous forme d'une poudre fine cristalline. Elles permettent notamment avantageusement, d'une part, d'éviter toute dégradation du sel d'intérêt, et, d'autre part, d'éviter la formation de granules du sel d'intérêt, qui ne pourraient être séchés à coeur qu'au prix d'une forte augmentation de température susceptible d'en entraîner la dégradation.

Dans des modes de mise en oeuvre particuliers de l'invention, il est réalisé, entre la première étape de séchage et la deuxième étape de séchage, une étape de séchage intermédiaire sous agitation, de préférence sous faible agitation, à une température comprise entre la première température T1 et la deuxième température T2, de préférence entre 30 et 40 °C et préférentiellement d'environ 32 °C, et :

- à une pression comprise entre 200 et 300 mbar, de préférence sensiblement égale à la première pression P1, pendant une durée comprise entre 60 et 100 minutes, de préférence d'environ 75 minutes comprenant une durée de 15 minutes de montée en température,

- puis à une pression comprise entre 10 et 30 mbar, de préférence sensiblement égale à la deuxième pression P2, pendant une durée comprise entre 60 et 100 minutes, de préférence d'environ 60 minutes.

Le procédé répondant à de telles caractéristiques permet avantageusement d'obtenir un sel d'acide acétylsalicylique et d'un acide aminé basique, notamment de l'acétylsalicylate de lysine, qui répond aux spécifications de la pharmacopée, en particulier en termes de faibles teneurs résiduelles en eau et en solvants, et de faibles teneurs en impuretés, notamment en acide salicylique.

Le procédé selon l'invention est de préférence mis en oeuvre par lots. Préférentiellement, l'ensemble de ses étapes sont réalisées sous atmosphère inerte. Dans des modes de mise en oeuvre particuliers de l'invention, elles sont en outre réalisées en conditions stériles.

Le procédé selon l'invention est avantageusement facile et rapide à mettre en oeuvre, qui plus est à faible coût. Tout dispositif classique en lui-même peut être utilisé à cet effet, en particulier les dispositifs conventionnels pour la production de principes actifs pharmaceutiques à l'échelle industrielle.

En particulier, les étapes de filtration, lavage et séchage du sel d'acide acétylsalicylique et de l'acide aminé basique peuvent être réalisées dans un seul et même appareillage, tel qu'un filtre sécheur muni de moyens d'agitation pilotés.

Selon un autre aspect, il est décrit une composition obtenue par un procédé selon l'invention répondant à l'une ou plusieurs des caractéristiques ci-avant.

Cette composition comporte notamment une teneur en sel d'acide acétylsalicylique comprise entre 97 et 100 %, notamment comprise entre 98 et 100 %. Elle présente une teneur en eau inférieure à 0,3 %, et une teneur en acide salicylique inférieure à 0,3 %. Conditionnée de manière hermétique sous atmosphère inerte, elle reste stable pendant au moins 6 mois.

Cette composition peut notamment être utilisée pour la fabrication d'une formulation pharmaceutique la comprenant en quantité efficace dans un véhicule pharmaceutiquement acceptable, le cas échéant en association avec d'autres principes actifs, et tout additif classique en lui-même.

Elle trouve notamment des applications en tant que principe actif à effet antalgique, antipyrétique, anti-inflammatoire, antirhumatismal, inhibiteur de l'agrégation plaquettaire, etc., pour le traitement curatif et/ou préventif de pathologies diverses, telles que les rhumatismes, les névralgies, les myalgies, les migraines, les maladies cardiovasculaires et cérébrovasculaires, etc. Il est aussi décrit un sel d'acide acétylsalicylique et d'un acide aminé basique, notamment de lysine, se présentant sous une forme granulaire, plus précisément sous forme d'une poudre de cristaux, et susceptible d'être obtenu par mise en oeuvre d'un procédé selon l'invention, répondant à l'une ou plusieurs des caractéristiques ci-avant, comprenant une étape de recristallisation. Cette poudre se caractérise par une teneur en sel d'acide acétylsalicylique comprise entre 97 et 100 %, une teneur en eau inférieure à 0,3 % et une teneur en acide salicylique inférieure à 0,3 %.

Elle présente en outre un diamètre moyen des grains compris entre 100 et 110 μm, mesuré par exemple par un dispositif Malvern dans des conditions opératoires normales, et une homogénéité de taille des cristaux élevée. Les cristaux présentant une telle taille présentent notamment avantageusement une surface spécifique relativement faible, de sorte qu'est minimisée la possibilité d'absorption éventuelle, à leur surface, de solvants résiduels préjudiciables à la stabilité du produit lors de sa conservation.

Les caractéristiques et avantages du procédé selon l'invention apparaîtront plus clairement à la lumière de l'exemple de mise en oeuvre ci-après, fourni à simple titre illustratif et nullement limitatif de l'invention, avec l'appui des figures 1a à 7, dans lesquelles :

- la figure 1a montre un chromatogramme HPLC obtenu pour une solution témoin d'acide acétylsalicylique à une concentration de 0,1 mg/ml ;
- la figure 1b montre un chromatogramme HPLC obtenu pour une solution d'acétylsalicylate de DL-lysine obtenu par un procédé conforme à l'invention, à une concentration de 0,18 mg/ml ;
- la figure 2a montre un chromatogramme HPLC obtenu pour une solution témoin d'acide salicylique à une concentration de 0,03 mg/ml ;
- la figure 2b montre un chromatogramme HPLC obtenu pour une solution d'acétylsalicylate de DL-lysine obtenu par un procédé conforme à l'invention, avant séchage, à une concentration de 10 mg/ml ;
- la figure 2c montre un chromatogramme HPLC obtenu pour une solution d'acétylsalicylate de DL-lysine obtenu par un procédé conforme à l'invention, après séchage, à une concentration de 10 mg/ml ;
- la figure 3 est un graphe indiquant la taille et la distribution de taille des particules d'une poudre d'acétylsalicylate de DL-lysine obtenue par un procédé conforme à l'invention, après séchage ;
- la figure 4 montre une image obtenue par microscopie de particules de la poudre de la figure 3 ;
- la figure 5 montre un chromatogramme HPLC obtenu pour une solution d'acétylsalicylate de DL-lysine obtenu par un procédé conforme à l'invention, comprenant une étape de recristallisation ;
- la figure 6 est un graphe indiquant la taille et la distribution de taille des particules d'une poudre d'acétylsalicylate de DL-lysine recristallisée obtenue par un procédé conforme à l'invention, après séchage ;
- et la figure 7 montre une image obtenue par microscopie de particules de la poudre de la figure 6.

<u>EXEMPLE 1</u> - Procédé de synthèse d'acétylsalicylate de DL-lysine

On prépare de l'acétylsalicylate de DL-lysine (III), à partir d'acide acétylsalicylique (I) et de DL-lysine (II), selon le schéma réactionnel :

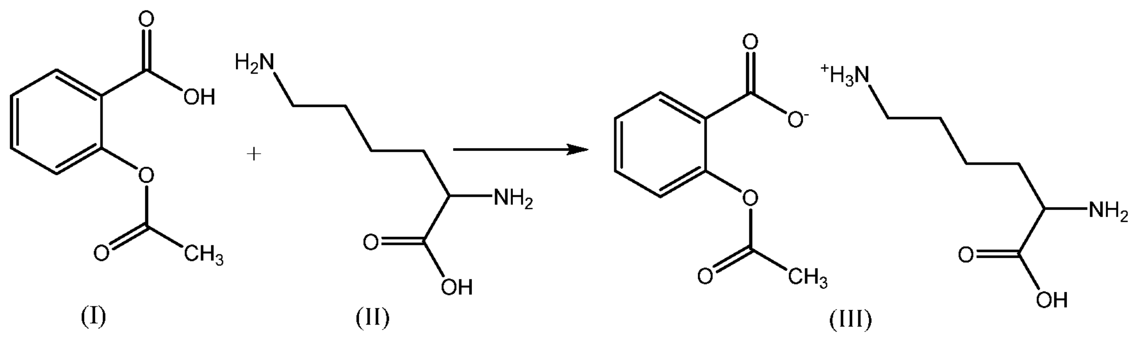

(I) (II) (III)

par le procédé conforme à l'invention décrit ci-après.

Préparation des solutions de réactifs

Les réactifs de départ sont l'acide acétylsalicylique et la DL-lysine.

Une solution d'acide acétylsalicylique à 17,7 % (p/p) dans l'acétone est préparée, à une température comprise entre 10 et 30 °C, dans un premier contenant dans lequel est appliqué un balayage d'azote. A cet effet, 400 g d'acide acétylsalicylique, sous forme de poudre blanche, puis 1 856 g d'acétone, sont introduits dans le contenant. Le mélange est agité, à 450 tr/min, jusqu'à dissolution complète de l'acide acétylsalicylique. On obtient une solution limpide et incolore, nommée ci-après Solution A. Cette solution présente une masse volumique à 20 °C comprise entre 0,850 et 0,860 g/cm$^3$.

Par ailleurs, une solution aqueuse de DL-lysine à 33 % (p/p) dans l'eau est préparée dans un deuxième contenant, à une température comprise entre -10 et 40 °C. A cet effet, 600 g d'une solution de DL-lysine à 50 % dans l'eau sont mélangés à 308,85 g d'eau osmosée. Le mélange est soumis à agitation jusqu'à obtention d'une solution claire et limpide, nommée ci-après Solution L. Cette solution présente une masse volumique à 20 °C comprise entre 1,080 et 1,090 g/cm$^3$.

Mélange des solutions de réactifs et mûrissement des cristaux

Le réacteur de synthèse mis en oeuvre pour la formation de l'acétylsalicylate de DL-lysine est un réacteur double enveloppe sous balayage d'azote, équipé d'une agitation double étage et relié à un cryothermostat.

On utilise, pour l'introduction des solutions de réactifs dans le réacteur, des pompes doseuses IKA. Ces pompes sont réglées, en fréquence et pourcentage de course du piston, pour présenter des débits respectifs de 13,07 l/h pour la Solution A, et de 4,47 l/h pour la Solution L. Ces débits respectifs permettent l'introduction simultanée dans le réacteur de quantités équimolaires d'acide acétylsalicylique et de DL-lysine (à raison de 0,182 mol.min$^{-1}$ de chacun des réactifs). Les fréquences des pompes doseuses sont réglées de manière sensiblement identiques l'une à l'autre, pour assurer que les quantités des réactifs introduites dans le réacteur permettent le maintien de quantités équimolaires des réactifs dans le réacteur pendant tout le temps du mélange.

Préalablement à l'introduction de la Solution A et de la Solution L dans le réacteur, le cryothermostat est mis en marche pour assurer la circulation, dans la double enveloppe du réacteur, d'un fluide réfrigérant à une température inférieure ou égale à -15 °C.

Les pompes doseuses sont ensuite actionnées en simultané, pour introduire la Solution A et la Solution L dans le réacteur, dans un ratio des deux réactifs qui est équimolaire à chaque instant t.

Durant cette étape, le milieu réactionnel est soumis à agitation, à une première vitesse d'agitation relativement faible, comprise entre 240 et 450 tr/min, de sorte à ne pas casser les cristaux qui se forment.

Durant ces étapes, la température du milieu réactionnel est maintenue comprise entre -10 °C et 15 °C. Plus précisément, elle est mesurée à 10,1 °C. La réaction de formation du sel s'effectue au fur et à mesure de l'introduction des solutions de réactifs dans le réacteur.

Lorsque le volume du milieu réactionnel atteint 2,5 l, le fonctionnement des pompes doseuses est interrompu.

Il a été introduit dans le réacteur : 1.708 l de Solution A et 0.584 l de Solution L.

Le milieu réactionnel contenu dans le réacteur est refroidi à -10 °C sous agitation réduite, à une vitesse d'agitation de 360 tr/min. Le milieu réactionnel est maintenu sous cette agitation et à -10 °C pendant 1 h.

Filtrations et lavage

Le milieu réactionnel est transféré dans un filtre sécheur agitateur.

Ce filtre sécheur est équipé d'une double enveloppe reliée à un cryothermostat, pour le contrôle de la température en son intérieur.
Le milieu réactionnel est soumis à une première étape de filtration sous pression réduite de 270 mbar, sous agitation. Cette filtration est réalisée en plusieurs séquences, de sorte à réaliser un empâtage puis un lissage du produit contenu dans le filtre. Après environ 30 min, on obtient dans le filtre un produit homogène, facilement agitable.
Le produit est soumis à un lavage par du propanol-2, dont il est introduit dans le filtre un volume correspondant à 1,5 fois la masse d'acétylsalicylate de DL-lysine obtenue, soit 501,50 g de propanol-2.
Le mélange obtenu est soumis à agitation pendant 10 min, à une vitesse d'agitation de 360 tr/min et sous pression réduite, pour obtenir une suspension homogène.
Ce mélange est ensuite soumis à une deuxième étape de filtration sous pression réduite de 270 mbar, sous agitation. Cette filtration est réalisée en plusieurs séquences, de sorte à réaliser un empâtage puis un lissage du produit contenu dans le filtre. Après environ 60 min, on obtient dans le filtre un produit homogène, facilement agitable.
Un échantillon de ce produit est prélevé et analysé pour sa composition, comme décrit de manière détaillée plus avant dans la présente description.

Séchage

Le séchage du produit de réaction est effectué en 3 phases distinctes, comme détaillé ci-après.
L'ensemble de ces étapes sont réalisées sous agitation, dans le filtre sécheur, dans lequel une pression réduite est appliquée par une tubulure connectée au niveau du couvercle, au-dessus du produit à sécher.

Phase 1 :

Dans la phase 1, la pression appliquée dans le filtre sécheur est d'environ 240 mbar, et la température est de 22 °C. La vitesse d'agitation est de 10 tr/min. Cette séquence a une durée de 135 min, dont 15 min de montée en température jusqu'à 22 °C, et 120 min de maintien à cette température.

Phase 2 :

Dans la phase 2, la température est augmentée, pendant une durée de 15 min, jusqu'à parvenir à une valeur de 32 °C. L'agitation est maintenue à 10 tr/min. La pression est maintenue à 240 mbar pendant 60 min, puis à une valeur inférieure ou égale à 20 mbar pendant 60 min supplémentaires.

Phase 3 :

La température est augmentée, pendant une durée de 15 min, jusqu'à parvenir à une valeur de 42 °C. L'agitation est maintenue à 10 tr/min, et la pression à une valeur inférieure ou égale à 20 mbar pendant 120 min.
On obtient au total 427,88 g (rendement 92,4 %) d'un produit sec qui se présente sous forme d'une poudre fine cristalline blanche, nommé ci-après ASL. Un échantillon de ce produit sec est prélevé dans le filtre, sous atmosphère inerte, et soumis à analyse, comme détaillé ci-après.
L'acétylsalicylate de DL-lysine ainsi obtenu est conditionné en flacon hermétique sous balayage d'azote. Il est conforme aux spécifications de la pharmacopée, pour une mise en oeuvre dans le domaine pharmaceutique.

EXEMPLE 2 - Analyse du produit obtenu

2.1/ Analyse par HPLC - Teneurs en acide acétylsalicylique et en acide salicylique

L'analyse par HPLC est réalisée selon la méthode de la Pharmacopée Européenne 2.2.29, comme indiqué ci-dessous.
A cet effet, il est mis en oeuvre les conditions opératoires suivantes.

Pour le dosage de l'acide acétylsalicylique

Colonne : Luna C18 (Référence : 00G-4041-E0)
Longueur : 250 mm
Diamètre intérieur : 4,6 mm

Granulométrie : 5 μm
Phase mobile : Acétonitrile : 400 V
Eau : 600 V
Acide orthophosphorique 85 % : 2 V
Débit : 1,0 ml/min
Température : 25 °C
Volume injecté : 10 μl
Longueur d'onde : 237 nm
Durée de l'analyse : 15 minutes
Temps de rétention de l'acide acétylsalicylique : environ 5,4 min

Une solution témoin est préparée par dissolution de 50,0 mg d'acide acétylsalicylique dans 50 ml de phase mobile, puis dilution de 5 ml de la solution obtenue dans 50 ml de phase mobile.

Les solutions des échantillons d'ASL à tester pour leur teneur en acide acétylsalicylique sont préparées par dissolution de 90,6 mg d'échantillon dans 50 ml de phase mobile, puis dilution de 5 ml de la solution obtenue dans 50 ml de phase mobile.

La teneur de chaque échantillon d'ASL en acide acétylsalicylique est obtenue par l'équation suivante :

$$T = \frac{Pt}{Pe} \times \frac{T}{100} \times \frac{Ae}{At} \times \frac{100 - Ht}{100 - He} \times \frac{326{,}3}{180{,}16} \times 100$$

où :

Ae représente l'aire du pic d'acide acétylsalicylique dans le chromatogramme de la solution d'échantillon à tester
At représente l'aire du pic d'acide acétylsalicylique dans le chromatogramme de la solution témoin
Pt représente le poids de l'acide acétylsalicylique dans la solution témoin en mg
T représente le titre de l'acide acétylsalicylique en %
Pe représente le poids de la solution d'échantillon en mg
Ht représente la perte halogène du témoin en %
He représente la perte halogène de la matière première en %

Les chromatogrammes obtenus, pour l'acide acétylsalicylique et pour l'ASL sec obtenu selon l'Exemple 1 ci-avant, sont montrés respectivement sur les figures 1a et 1b. On en déduit un titre en acide acétylsalicylique de 98,3 %. Cette valeur est conforme aux spécifications de la Pharmacopée Européenne (2.2.29), qui requiert un titre compris entre 97,0 et 101,0 %.

Pour le dosage de l'acide salicylique (impureté)

Les conditions opératoires sont identiques à celles indiquées ci-avant pour le dosage de l'acide acétylsalicylique, à l'exception de :

Phase mobile : acide orthophosphorique 85% : 4 V
Durée de l'analyse : 30 min pour les essais et 15 min pour les témoins
Temps de rétention : acide salicylique : environ 8,3 min
acide acétylsalicylique : environ 5,8 min

Une solution témoin d'acide salicylique à 0,3 % est préparée par dissolution de 150,0 mg d'acide salicylique dans 50 ml de phase mobile, puis dilution de 1 ml de la solution obtenue dans 100 ml de phase mobile.

Les solutions des échantillons d'ASL à tester pour leur teneur en acide acétylsalicylique sont préparées par dissolution de 100 mg d'échantillon dans 10 ml de phase mobile.

La teneur de chaque échantillon d'ASL en acide salicylique est obtenue par l'équation suivante :

$$T = \frac{Pt \, x \, T}{100} \times \frac{Ae}{At} \times \frac{1}{Pe} \times \frac{1 - Ht}{1 - He} \times \frac{10}{5000} \times 100$$

où :

Ae représente l'aire du pic d'acide salicylique dans le chromatogramme de la solution d'échantillon à tester
At représente l'aire du pic d'acide salicylique dans le chromatogramme de la solution témoin
Pt représente le poids de l'acide salicylique dans la solution témoin en mg
T représente le titre de l'acide salicylique en %
Pe représente le poids de la solution d'échantillon en mg
Ht représente la perte halogène du témoin en %
He représente la perte halogène de la matière première en %

Les chromatogrammes obtenus, pour l'acide salicylique, pour l'ASL avant séchage et pour l'ASL sec obtenus selon l'Exemple 1 ci-avant, sont montrés respectivement sur les figures 2a, 2b et 2c. On en déduit un titre en acide salicylique de 0,08 % pour l'ASL avant séchage, et de 0,13 % pour l'ASL sec. Ces valeurs sont conformes aux spécifications de la Pharmacopée Européenne (2.2.29), qui requiert un titre en acide salicylique inférieur ou égal à 0,3 %.

2.2/ Autres analyses

Les autres analyses ci-après sont également effectuées sur l'ASL sec obtenu dans l'Exemple 1.

Protocoles opératoires

- Humidité résiduelle : perte à la dessiccation au dessiccateur halogène 5 g / 90 °C / 20 min.

- Teneur en eau : selon la Pharmacopée Européenne 2.5.12, dosage réalisé par Karl Fisher sur 1 g d'ASL.

- Cendres sulfuriques : selon la Pharmacopée Européenne 2.4.14, sur 1,0 g d'ASL dans un creuset de masse $m_{creuset}$ (masse totale initiale du creuset et de l'ASL : $m_{essai}$), avec 1 mL du réactif acide sulfurique R, chauffage sur plaques chauffantes jusqu'à carbonisation complète, pendant environ 4 h, puis, dans un four, montée en température jusqu'à 600 °C en 2 h et maintien à 600 °C pendant 30 min. Après refroidissement, peser (masse totale finale du creuset et de l'échantillon : $m_{final}$). Le % en résidu est obtenu par l'équation suivante :

$$\% \text{ résidu} = \frac{m_{final} - m_{creuset}}{m_{essai}} \text{ x } 100$$

- Chlorure : selon la Pharmacopée Européenne 2.4.4, avec les réactifs : acide nitrique dilué R, solution de nitrate d'argent R2, solution mère de chlorures.

Il est préparé une solution à 5 ppm de chlorure (R) par dilution de 1 mL de solution mère de chlorures dans 100 ml d'eau R.
La solution à tester est préparée par dissolution de 0,33 g d'ASL dans 15 mL d'eau R. Il est ajouté 1 mL d'acide nitrique dilué R et ce mélange est versé en une seule fois dans un tube à essai contenant 1 mL de solution de nitrate d'argent R2.
La solution témoin est préparée de la même manière, en utilisant un mélange de 10 mL de solution à 5 ppm de chlorure R et 5 mL d'eau R.
Un blanc est préparé dans les mêmes conditions avec 15 mL d'eau R.
Après 5 min à l'abri de la lumière, l'opalescence de la solution à tester est examinée et comparée à celle du témoin.

- Métaux lourds : selon la Pharmacopée Européenne 2.4.8, procédé A, avec les réactifs : solution tampon pH 3,5 R, solution de thioacétamide R à 4 %, réactif à la glycérine, solution mère de plomb

Le réactif à la glycérine est obtenu par mélange de 5 mL d'eau R, 15 mL d'hydroxyde de sodium 1M et 20 mL de glycérol à 85% R.
Le réactif au thioacétamide R est obtenu par mélange de 0,2 mL de solution au thioacétamide R et 1 mL de réactif à la glycérine.
La solution à 2 ppm de plomb est préparée par dilution de 0,2 mL de solution mère de plomb dans 100 mL d'eau R.
La solution à tester est préparée par dissolution de 4,0 g d'ASL dans 20 mL d'eau R.

La solution témoin est préparée par mélange de 10 mL de solution à 2 ppm de plomb et 2 mL de solution à tester.

Un blanc est préparé dans les mêmes conditions avec 10 mL d'eau R et 2 mL de solution à tester.

A chaque solution, sont mélangés 2 mL de solution tampon pH 3,5 R, puis 1,2 mL de réactif au thioacétamide R.

Après 5 min à l'abri de la lumière, l'opalescence de la solution à tester est examinée et comparée à celle du témoin et à celle du blanc.

- pH : selon la Pharmacopée Européenne 2.2.3, une solution est reconstituée par dissolution de 1 g d'ASL dans 10 ml d'eau exempte de dioxyde de carbone, avec agitation pendant 30 s. Son aspect est évalué et son pH est mesuré.
- Teneur en chaque impureté inconnue : par dosage HPLC, selon le protocole opératoire décrit ci-avant. La teneur en chaque impureté est déterminée par l'équation suivante :

$$T = \frac{PtASL\,x\,T}{100} \text{ x } \frac{Ae}{A\,tASL} \text{ x } \frac{1}{Pe} \text{ x } \frac{1-Ht}{1-He} \text{ x } \frac{10}{5000} \text{ x } 100$$

où :

Ae représente l'aire du pic de l'impureté dans le chromatogramme de la solution d'échantillon à tester
AtASL représente l'aire du pic d'ASL dans le chromatogramme de la solution témoin à 0,1 %
PtASL représente le poids d'ASL dans la solution témoin en mg
T représente le titre de l'ASL en %
Pe représente le poids de la solution d'échantillon en mg
Ht représente la perte halogène du témoin d'acide salicylique en %
He représente la perte halogène de la matière première en %

- Solvants résiduels par HS-CPG, selon la Pharmacopée Européenne 2.2.28. Les conditions opératoires sont les suivantes :

Conditions Espace de tête :

Température du four : 80 °C
Température de la boucle : 80 °C
Température de la ligne de transfert : 85 °C
Cycle de la CPG : 20 min
Temps d'équilibrage du flacon : 15 min
Temps de pressurisation : 0,5 min
Temps de remplissage de la boucle : 0,04 min
Temps d'équilibrage de la boucle : 0,5 min
Temps d'injection : 0,5 min
Volume du flacon : 20 mL
Volume d'échantillon : 5 mL

Conditions Chromatographiques :

Colonne : DB-FFAP (Référence : 122-3232)
Longueur:30 m
Diamètre intérieur : 250 µm
Epaisseur du film : 0,25 µm

Gaz Vecteur : Hélium
Débit : 1,50 ml/min
Température de l'injecteur : 150 °C
Mode : Split
Split Ratio : 1/2
Température du four : 80 °C pendant 2,30 min, puis rampe de 30 °C/min jusqu'à une température de 250 °C, pendant un
temps total de 7,79 min
Température du détecteur (FID) : 300 °C

Débit $H_2$ : 45 mL/min
Débit $O_2$ : 400 mL/min
Constant Makeup : 30,0 mL/min
Durée de l'analyse : 8,0 min
Temps de rétention : acétone: environ 1,7 min
isopropanol : environ 1,9 min

Une solution témoin d'acétone à 5000 ppm est préparée par dissolution de 500,0 mg d'acétone dans 50 ml d'eau puis dilution de 5 ml de cette solution dans 100 ml d'eau.

Une solution témoin d'isopropanol à 5000 ppm est préparée par dissolution de 500,0 mg d'isopropanol dans 50 ml d'eau puis dilution de 5 ml de cette solution dans 100 ml d'eau.

Une solution témoin d'éthanol à 5000 ppm est préparée par dissolution de 500,0 mg d'éthanol dans 50 ml d'eau puis dilution de 5 ml de cette solution dans 100 ml d'eau.

La solution à tester est préparée par dissolution de 2 g d'ASL dans 20 ml d'eau.

La teneur en solvant dans l'ASL est déterminée par l'équation :

$$T = \frac{Pt \, x \, T}{100} \text{ x } \frac{Ae}{At} \text{ x } \frac{1}{Pe} \text{ x } \frac{20}{1000} \text{ x } 10^6$$

où :

Ae représente l'aire du pic de solvant dans le chromatogramme de la solution d'échantillon à tester

At représente l'aire du pic de solvant dans le chromatogramme de la solution témoin à 0,1 %

Pt représente le poids de solvant dans la solution témoin en mg

T représente le titre de solvant en %

Pe représente le poids de la solution d'échantillon en mg

Résultats

Les résultats obtenus, pour l'ASL sec obtenu à l'Exemple 1, sont indiqués dans le tableau 1 ci-après. Les spécifications de la Pharmacopée Européenne sont également indiquées dans ce tableau.

Tableau 1 - Résultat d'analyses de l'acétylsalicylate de DL-lysine obtenu par un procédé selon un mode de mise en oeuvre particulier de l'invention

| Test | Spécifications | ASL |
|---|---|---|
| Aspect de la poudre | Cristalline blanche et fine | Conforme |
| Aspect de la solution reconstituée | Solution transparente | Conforme |
| pH de la solution reconstituée | 4,5 à 6,5 | 5,9 |
| Teneur en eau | ≤ 0,5 % | 0,4% |
| Humidité résiduelle | ≤ 1,0 % | 0,4% |
| Impuretés<br>Autre impureté inconnue<br>Somme des impuretés<br>Solvants résiduels | <br>≤ 0,1 %<br>-<br>≤ 0,5% | <br>0,02 %<br>0,15%<br>0,19 % |

Il ressort de ces résultats, combinés à ceux obtenus pour le dosage HPLC des teneurs en acide acétylsalicylique et en acide salicylique indiqués ci-avant, que le procédé conforme à la présente invention permet d'obtenir, avec un rendement élevé, un sel d'acétylsalicylique et de DL-lysine de haut degré de pureté, et conforme aux spécifications de la Pharmacopée Européenne.

2.3/ Taille des particules

La taille des particules a été analysée au moyen d'un appareil Scirocco 2000 (Malvern), selon un protocole classique en lui-même.
Les résultats obtenus, en termes de taille des particules, sont montrés sur la figure 3. La taille moyenne des particules est de 6,931 μm.
Une image obtenue par microscopie est montrée sur la figure 4.

EXEMPLE 3 - Etudes de stabilité

3.1/ Expérience 1

Une étude de stabilité du produit obtenu à l'Exemple 1 est effectuée par stockage du produit sec, sous atmosphère inerte et à l'abri de la lumière, à une température de 5 °C, pendant une durée de 3 mois.
Aux temps T = 0, T = 1 mois et T = 3 mois, un échantillon de produit est prélevé et soumis à l'ensemble des tests décrits dans l'Exemple 2.
A titre d'exemple comparatif, la même expérience est réalisée sur un produit obtenu par le procédé de préparation de l'art antérieur décrit dans le document WO 2011/039432, prévoyant notamment un défaut molaire en DL-lysine (plus précisément, avec un rapport molaire acide acétylsalicylique / DL-lysine égal à 1/0,89).
Les résultats obtenus sont montrés dans le tableau 2 ci-après.

Tableau 2 - Résultats de tests de stabilité pour un produit obtenu par un procédé conforme à l'invention et par le procédé décrit dans le document WO 2011/039432

| Test | Spécifications | Procédé conforme à l'invention | | | Procédé du WO 2011/039432 | | |
|---|---|---|---|---|---|---|---|
| Temps (Mois) | | 0 | 1 | 3 | 0 | 1 | 3 |
| pH de la solution reconstituée | 4,5 à 6,5 | 5,9 | 5,6 | 5,5 | 5,2 | 5,2 | 5,1 |
| Teneur en eau (%) | ≤ 0,5 | 0,4 | 0,5 | 0,3 | - | - | - |
| Humidité résiduelle (%) | ≤ 1,0 | 0,4 | 0,6 | 0,6 | 0,72 | 0,61 | 0,61 |
| Teneur HPLC en acide acétylsalicylique (%) | 97,0 à 101,0 | 98,3 | 98,5 | 98,8 | 93,6 | 90,0 | 91,6 |
| Impuretés (%) | | | | | | | |
| Acide salicylique | ≤ 0,3 | 0,13 | 0,17 | 0,21 | 1,9 | 1,9 | 2,0 |
| Autre impureté inc. | ≤ 0,1 | 0,02 | 0,02 | 0,02 | - | 0,026 | 0,027 |
| Somme des impuretés | - | 0,15 | 0,22 | 0,25 | - | 0,09 | 0,07 |

Pour l'ensemble des conditions testées, la poudre présente un aspect conforme (poudre cristalline blanche et fine), de même que la solution reconstituée (solution transparente).
Comme on peut le constater, le produit obtenu par le procédé conforme à l'invention de l'Exemple 1 reste stable dans le temps, pendant toute la durée de l'expérience, et conforme aux spécifications de la pharmacopée, notamment en termes de teneurs en acide acétylsalicylique et en acide salicylique, qui en est le principal produit de dégradation.
En comparaison, le produit obtenu par un procédé de l'art antérieur présente une stabilité dans le temps nettement inférieure. Le degré de pureté initial du produit n'est en outre pas satisfaisant.

3.2/ Expérience 2

Une étude de stabilité du produit obtenu à l'Exemple 1 (nouveau lot de synthèse) est effectuée par stockage du produit sec, sous atmosphère inerte et à l'abri de la lumière, à une température de 5 °C, pendant une durée de 6 mois.
Aux temps T = 0, T = 1 mois, T = 3 mois et T = 6 mois, un échantillon de produit est prélevé et soumis à l'ensemble des tests décrits dans l'Exemple 2.
Les résultats obtenus sont indiqués dans le Tableau 3 ci-après.

Tableau 3 - Résultats de tests de stabilité pour un produit obtenu par un procédé conforme à l'invention

| Temps (Mois) | Spécifications | 0 | 1 | 3 | 6 |
|---|---|---|---|---|---|
| Aspect de la poudre | Cristalline blanche / fine | C | C | C | C |
| Aspect de la solution reconstituée | Transparente | C | C | C | C |
| pH de la solution reconstituée | 4,5 à 6,5 | 5,7 | 5,8 | 5,4 | 6,3 |
| Teneur en eau (%) | ≤ 0,5 | 0,4 | 0,3 | 0,3 | 0,3 |
| Humidité résiduelle (%) | ≤ 1,0 | 0,7 | 0,5 | 0,6 | 0,6 |
| Teneur HPLC acide acétylsalicylique (%) | 97,0 à 101,0 | 97,5 | 97,5 | 98,1 | 97,5 |
| Impuretés (%)<br>Acide salicylique<br>Autre impureté inc.<br>Somme des impuretés | <br>≤ 0,3<br>≤ 0,1<br> | <br>0,28<br>0,03<br>0,32 | <br>0,28<br>0,03<br>0,33 | <br>0,29<br>0,03<br>0,33 | <br>0,30<br>0,03<br>0,34 |
| C = conforme | | | | | |

Comme on peut le constater, le produit obtenu par le procédé conforme à l'invention de l'Exemple 1 reste stable dans le temps et conforme aux spécifications de la pharmacopée, pendant toute la durée de l'expérience, y compris après 6 mois de stockage.

Les légers écarts constatés par rapport à l'Expérience 1, pour le même produit, sont imputables aux incertitudes liées aux méthodes de dosage, et ne sont pas significatifs.

EXEMPLE 4 - Exemples comparatifs

Le procédé de synthèse décrit à l'Exemple 1 est mis en oeuvre, mais dans des rapports molaires acide acétylsalicylique / DL-lysine initiaux différents, plus précisément des rapports molaires respectivement de 1/0,977 (exemple comparatif C1) et 1/1,034 (exemple comparatif C2), très proches de ceux préconisés par l'art antérieur.

Un procédé conforme à l'invention, avec un rapport molaire acide acétylsalicylique / DL-lysine de 1/1, est mis en oeuvre en parallèle (Exemple Inv.).

Les conditions opératoires mises en oeuvre sont strictement identiques, à l'exception du débit des pompes. Pour chacun des exemples, ces débits des pompes sont indiqués dans le tableau 4 ci-après.

Tableau 4 - Conditions opératoires de synthèse des produits comparatifs C1 et C2, et du produit selon l'invention Inv.

| Exemple | Débit pompe Solution A (l/h) | Débit pompe Solution B (l/h) | Rapport molaire acide acétylsalicylique / lysine |
|---|---|---|---|
| Inv. | 13,07 | 4,47 | 1/1 |
| C1 | 13,07 | 4,37 | 1/0,977 |
| C2 | 13,07 | 4,62 | 1/1,034 |

Après séchage, on obtient, pour chaque exemple, les quantités de produit sec indiquées dans le tableau 5 ci-après.

Un échantillon de chacun de ces produits est soumis à analyse HPLC, comme décrit dans l'Exemple 2 ci-avant, pour déterminer sa teneur en acide acétylsalicylique. Les résultats obtenus sont indiqués dans le tableau 5 ci-après.

Tableau 5 - Résultats d'analyse des produits obtenus, pour un produit conforme à l'invention Inv. et des produits comparatifs C1 et C2

| Exemple | Quantité de produit sec obtenu (g) | Teneur HPLC en acide acétylsalicylique (%) | Teneur HPLC en acide salicylique (%) |
|---|---|---|---|
| Inv. | 427,88 | 98,3 | 0,13 |
| C1 | 351,86 | 92,2 | 1,4 |

(suite)

| Exemple | Quantité de produit sec obtenu (g) | Teneur HPLC en acide acétylsalicylique (%) | Teneur HPLC en acide salicylique (%) |
|---|---|---|---|
| C2 | 437,41 | 90 | 1,7 |

Comme on peut le constater, les données HPLC démontrent clairement que les produits comparatifs C1 et C2, obtenus à partir de rapports molaires différents du rapport molaire 1/1 préconisé par la présente invention, bien que très proches, présentent une teneur en acide acétylsalicylique bien inférieure à celle du produit obtenu conformément à la présente invention, et une teneur en l'impureté majoritaire, l'acide salicylique, largement supérieure. Ces produits comparatifs ne sont pas conformes, en terme de pureté, aux spécifications de la pharmacopée.

EXEMPLE 5 - Synthèse d'acétylsalicylate de DL-lysine avec étape de recristallisation

5.1/ Procédé de synthèse

On prépare de l'acétylsalicylate de DL-lysine (III), à partir d'acide acétylsalicylique (I) et de DL-lysine (II), comme décrit dans l'Exemple 1 ci-avant, à la différence près que, après la première étape de filtration, 220 g du produit obtenus sont soumis à une étape de recristallisation.
Cette étape est réalisée selon le protocole opératoire suivant :

- les 220 g de produit sont mis en suspension 440 ml de propan-2-ol ;
- 440 ml d'eau sont ajoutés au mélange, de sorte à solubiliser le produit,
- 880 ml de propan-2-ol sont ajoutés au milieu,
- la température du milieu est abaissée jusqu'à environ 25 °C, et la recristallisation est amorcée par ajouts de cristaux d'acétylsalicylate de lysine,
- la température du milieu est abaissée jusqu'à environ 5 °C, en environ 5 minutes.

A l'issue de ces opérations, des cristaux se sont formés dans le milieu.
Le milieu est soumis à une étape de filtration sous pression réduite de 270 mbar, puis à une étape de séchage, en 3 phases distinctes, suivant le protocole décrit dans l'Exemple 1 ci-avant.
On obtient au final 176,56 g (rendement de l'étape de recristallisation : 80,3 %) d'un produit sec qui se présente sous forme d'une poudre fine cristalline blanche. Un échantillon de ce produit sec est prélevé dans le filtre, sous atmosphère inerte, et soumis aux différentes analyses suivantes.

5.2/ Analyses

Les protocoles d'analyse sont identiques à ceux décrits dans l'Exemple 2 ci-avant.
La teneur en acide acétylsalicylique du produit solide obtenu est déterminée par HPLC. Le chromatogramme obtenu est montré sur la figure 5 (pic à 5,431 minutes). On en déduit un titre en acide acétylsalicylique de 98,5 %.
Les résultats obtenus pour les autres paramètres analysés sont indiqués dans le tableau 6 ci-après.

Tableau 6 - Résultat d'analyses de l'acétylsalicylate de DL-lysine obtenu par un procédé selon un mode de mise en oeuvre particulier de l'invention comportant une étape de recristallisation

| Test | ASL recristallisé |
|---|---|
| Aspect de la poudre | Blanche fine cristalline |
| Aspect de la solution reconstituée | Transparente limpide |
| Teneur en eau | 0,11 % |
| Teneur en acide salicylique | 0,24% |
| Teneur en autres impuretés | 0,05% |

Il ressort de ces résultats, combinés à celui obtenu pour le dosage HPLC de la teneur en acide acétylsalicylique indiqué ci-avant, que le procédé mis en oeuvre permet d'obtenir un sel d'acétylsalicylique et de DL-lysine de haut degré de pureté, et conforme aux spécifications de la Pharmacopée Européenne.

La taille des particules a en outre été analysée au moyen d'un appareil Scirocco 2000 (Malvern), selon un protocole classique en lui-même. Les résultats obtenus, en termes de taille des particules, sont montrés sur la figure 6. La taille moyenne des particules est de 104,219 μm.

Une image de la poudre d'ASL recristallisée, obtenue par microscopie, est montrée sur la figure 7.

Ces résultats montrent que la poudre d'acétylsalicylate de lysine recristallisée obtenue présente une distribution de taille resserrée, avec un diamètre moyen de particules de 104,219 μm.

## Revendications

1. Procédé de préparation d'un sel d'acide acétylsalicylique et d'un acide aminé basique, comprenant le mélange dans un réacteur d'une solution d'acide acétylsalicylique et d'une solution dudit acide aminé basique, à une température inférieure ou égale à 30 °C à pression atmosphérique, **caractérisé en ce que** ledit mélange est réalisé par introduction progressive dans le réacteur, simultanément, de ladite solution d'acide acétylsalicylique et de ladite solution dudit acide aminé basique, dans des conditions telles que durant toute la durée de ladite introduction, à chaque instant les quantités d'acide acétylsalicylique et dudit acide aminé basique introduites dans le réacteur sont équimolaires.

2. Procédé selon la revendication 1, comprenant une phase dite de mûrissement des cristaux, selon laquelle le milieu réactionnel, formé à l'issue de l'introduction de la solution d'acide acétylsalicylique et de la solution dudit acide aminé basique dans le réacteur, est maintenu sous agitation à une température comprise entre -15 °C et 20 °C, pendant une durée comprise entre 30 et 90 minutes.

3. Procédé selon l'une quelconque des revendications 1 à 2, selon lequel, à l'issue de la phase de mûrissement des cristaux, le solide contenu dans le milieu réactionnel est isolé, puis lavé au moyen d'un solvant organique.

4. Procédé selon l'une quelconque des revendications 1 à 2, selon lequel, à l'issue de la phase de mûrissement des cristaux, le solide contenu dans le milieu réactionnel est soumis à une étape de recristallisation du sel d'acide acétylsalicylique et dudit acide aminé basique, le cas échéant après avoir été isolé dudit milieu réactionnel, puis le cas échéant lavé au moyen d'un solvant organique.

5. Procédé selon la revendication 4, selon lequel ladite étape de recristallisation est réalisée dans un mélange de solvants comprenant au moins un alcool et de l'eau.

6. Procédé selon l'une quelconque des revendications 1 à 5, comprenant une étape finale de séchage du sel d'acide acétylsalicylique et dudit acide aminé basique obtenu, ledit séchage étant réalisé en au moins deux étapes successives, comprenant :

   - une première étape de séchage sous agitation, à une première pression comprise entre 200 et 300 mbar et à une première température comprise entre 20 et 25 °C, pendant une durée comprise entre 90 et 250 minutes ;
   - une deuxième étape de séchage sous agitation, à une deuxième pression comprise entre 10 et 30 mbar et à une deuxième température comprise entre 40 et 50 °C, pendant une durée comprise entre 90 et 250 minutes.

7. Procédé selon la revendication 6, selon lequel il est réalisé, entre la première étape de séchage et la deuxième étape de séchage, une étape de séchage intermédiaire sous agitation, à une température comprise entre la première température et la deuxième température, et à une pression comprise entre 200 et 300 mbar pendant une durée comprise entre 60 et 100 minutes, puis à une pression comprise entre 10 et 30 mbar pendant une durée comprise entre 60 et 100 minutes.

8. Procédé selon l'une quelconque des revendications 1 à 7, selon lequel la solution d'acide acétylsalicylique comprend de 0,8 à 0,9 mol/l d'acide acétylsalicylique, dans un solvant organique miscible à l'eau.

9. Procédé selon l'une quelconque des revendications 1 à 8, selon lequel la solution dudit acide aminé basique est une solution aqueuse comprenant de 4,5 à 5,5 mol/l dudit acide aminé basique.

10. Procédé selon l'une quelconque des revendications 1 à 9, selon lequel l'introduction progressive dans le réacteur

de la solution d'acide acétylsalicylique et de la solution dudit acide aminé basique est réalisée avec un débit d'introduction de la solution d'acide acétylsalicylique dans le réacteur compris entre 10 et 50 l/h, et un débit d'introduction de la solution dudit acide aminé basique dans le réacteur compris entre 2 et 15 l/h.

**11.** Procédé selon l'une quelconque des revendications 1 à 10, selon lequel l'acide aminé basique est choisi parmi la lysine, l'arginine, l'histidine et l'ornithine.

**12.** Procédé selon l'une quelconque des revendications 1 à 11, dont l'ensemble des étapes sont réalisées sous atmosphère inerte.

## Patentansprüche

**1.** Zubereitungsverfahren eines Salzes von Acetylsalicylsäure und einer basischen Aminosäure, umfassend das Mischen einer Acetylsalicylsäurelösung und einer Lösung der basischen Aminosäure in einem Reaktor bei einer Temperatur unterhalb von oder gleich 30 °C bei Atmosphärendruck, **dadurch gekennzeichnet, dass** das Mischen durch gleichzeitiges schrittweises Einbringen der Acetylsalicylsäurelösung und der Lösung der basischen Aminosäure in den Reaktor durchgeführt wird, unter Bedingungen derart, dass während der gesamten Dauer des Einbringens die Mengen an in den Reaktor eingebrachter Acetylsalicylsäure und der eingebrachten basischen Aminosäure zu jedem Zeitpunkt äquimolar sind.

**2.** Verfahren nach Anspruch 1, umfassend eine Phase der Kristallreifung, bei der die am Abschluss des Einbringens der Acetylsalicylsäurelösung und der Lösung der basischen Aminosäure in den Reaktor gebildeten Reaktionsumgebung unter Rühren bei einer Temperatur im Bereich zwischen -15 °C und 20 °C während einer Dauer im Bereich zwischen 30 und 90 Minuten gehalten wird.

**3.** Verfahren nach einem der Ansprüche 1 bis 2, wobei am Abschluss der Kristallreifungsphase der in der Reaktionsumgebung enthaltene Feststoff isoliert, anschließend mittels eines organischen Lösemittels gewaschen wird.

**4.** Verfahren nach einem der Ansprüche 1 bis 2, wobei am Abschluss der Kristallreifungsphase der in der Reaktionsumgebung enthaltene Feststoff einem Umkristallisierungsschritt des Salzes der Acetylsalicylsäure und der basischen Aminosäure unterzogen wird, gegebenenfalls nachdem er von der Reaktionsumgebung isoliert wurde, anschließend gegebenenfalls mittels eines organischen Lösemittels gewaschen wird.

**5.** Verfahren nach Anspruch 4, wobei der Umkristallisierungsschritt in einer Lösemittelmischung, umfassend mindestens einen Alkohol und Wasser, durchgeführt wird.

**6.** Verfahren nach einem der Ansprüche 1 bis 5, umfassend einen finalen Trocknungsschritt des erhaltenen Salzes der Acetylsalicylsäure und der basischen Aminosäure, wobei das Trocknen in mindestens zwei aufeinander folgenden Schritten durchgeführt wird, umfassend:

- einen ersten Trocknungsschritt unter Rühren, bei einem ersten Druck im Bereich zwischen 200 und 300 mbar und bei einer ersten Temperatur im Bereich zwischen 20 und 25 °C, während einer Dauer im Bereich zwischen 90 und 250 Minuten;
- einen zweiten Trocknungsschritt unter Rühren, bei einem zweiten Druck im Bereich zwischen 10 und 30 mbar und bei einer zweiten Temperatur im Bereich zwischen 40 und 50 °C, während einer Dauer im Bereich zwischen 90 und 250 Minuten.

**7.** Verfahren nach Anspruch 6, wobei zwischen dem ersten Trocknungsschritt und dem zweiten Trocknungsschritt ein zwischenliegender Trocknungsschritt unter Rühren durchgeführt wird, bei einer Temperatur im Bereich zwischen der ersten Temperatur und der zweiten Temperatur und bei einem Druck im Bereich zwischen 200 und 300 mbar während einer Dauer im Bereich zwischen 60 und 100 Minuten, anschließend bei einem Druck im Bereich zwischen 10 und 30 mbar und einer Dauer im Bereich zwischen 60 und 100 Minuten.

**8.** Verfahren nach einem der Ansprüche 1 bis 7, wobei die Acetylsalicylsäurelösung von 0,8 bis 0,9 mol/l Acetylsalicylsäure in einem mit Wasser mischbaren organischen Lösemittel umfasst.

**9.** Verfahren nach einem der Ansprüche 1 bis 8, wobei die Lösung der basischen Aminosäure eine wässrige Lösung

ist, umfassend von 4,5 bis 5,5 mol/l der basischen Aminosäure.

10. Verfahren nach einem der Ansprüche 1 bis 9, wobei das schrittweise Einbringen der Acetylsalicylsäurelösung und der Lösung der basischen Aminosäure in den Reaktor mit einem Einbringungsvolumenstrom der Acetylsalicylsäurelösung in den Reaktor im Bereich zwischen 10 und 50 l/h und einem Einbringungsvolumenstrom der basischen Aminosäure in den Reaktor im Bereich zwischen 2 und 15 l/h durchgeführt wird.

11. Verfahren nach einem der Ansprüche 1 bis 10, wobei die basische Aminosäure ausgewählt ist aus Lysin, Arginin, Histidin und Ornithin

12. Verfahren nach einem der Ansprüche 1 bis 11, wobei die Gesamtheit der Schritte unter inerter Atmosphäre durchgeführt wird.

## Claims

1. A process for preparing a salt of acetylsalicylic acid and a basic amino acid, which comprises mixing a solution of acetylsalicylic acid and a solution of said basic amino acid in a reactor, at a temperature of less than or equal to 30°C at atmospheric pressure, **characterized in that** said mixing is carried out by gradual introduction simultaneously of said solution of acetylsalicylic acid and of said solution of said basic amino acid into the reactor, under conditions such that, throughout said introduction, at each instant the amounts of acetylsalicylic acid and of said basic amino acid introduced into the reactor are equimolar.

2. The process as claimed in claim 1, comprising a "crystal maturation" phase, wherein the reaction medium, formed at the end of the introduction of the solution of acetylsalicylic acid and of the solution of said basic amino acid into the reactor, is kept with stirring at a temperature of between -15°C and 20°C, for a period of between 30 and 90 minutes.

3. The process as claimed in either one of claims 1 and 2, wherein, at the end of the crystal maturation phase, the solid contained in the reaction medium is isolated, then washed by means of an organic solvent.

4. The process as claimed in either one of claims 1 and 2, wherein, at the end of the crystal maturation phase, the solid contained in the reaction medium is subjected to a step of recrystallization of the salt of acetylsalicylic acid and said basic amino acid, where appropriate after having been isolated from said reaction medium, and then optionnally washed by means of an organic solvent.

5. The process as claimed in claim 4, wherein said recrystallization step is carried out in a mixture of solvents comprising at least one alcohol and water.

6. The process as claimed in any one of claims 1 to 5, comprising a final step of drying the salt of acetylsalicylic acid and said basic amino acid obtained, said drying being carried out in at least two successive steps, comprising:

- a first step of drying with stirring, at a first pressure of between 200 and 300 mbar and at a first temperature of between 20 and 25°C, for a period of between 90 and 250 minutes;
- a second step of drying with stirring, at a second pressure of between 10 and 30 mbar and at a second temperature of between 40 and 50°C, for a period of between 90 and 250 minutes.

7. The process as claimed in claim 6, wherein an intermediate drying step is carried out between the first drying step and the second drying step, said intermediate drying step being carried out with stirring, at a temperature between the first temperature and the second temperature, and at a pressure of between 200 and 300 mbar for a period of between 60 and 100 minutes, then at a pressure of between 10 and 30 mbar for a period of between 60 and 100 minutes.

8. The process as claimed in any one of claims 1 to 7, wherein the acetylsalicylic acid solution comprises from 0.8 to 0.9 mol/l of acetylsalicylic acid, in a water-miscible organic solvent.

9. The process as claimed in any one of claims 1 to 8, wherein the solution of said basic amino acid is an aqueous solution comprising from 4.5 to 5.5 mol/l of said basic amino acid.

**10.** The process as claimed in any one of claims 1 to 9, wherein the gradual introduction of the solution of acetylsalicylic acid and of the solution of said basic amino acid into the reactor is carried out with a flow rate of introduction of the acetylsalicylic acid solution into the reactor of between 10 and 50 l/h, and a flow rate of introduction of the basic amino acid solution into the reactor of between 2 and 15 l/h.

**11.** The process as claimed in any one of claims 1 to 10, wherein the basic amino acid is chosen from lysine, arginine, histidine and ornithine.

**12.** The process as claimed in any one of claims 1 to 11, all of the steps of which are carried out under an inert atmosphere.

FIG 1a

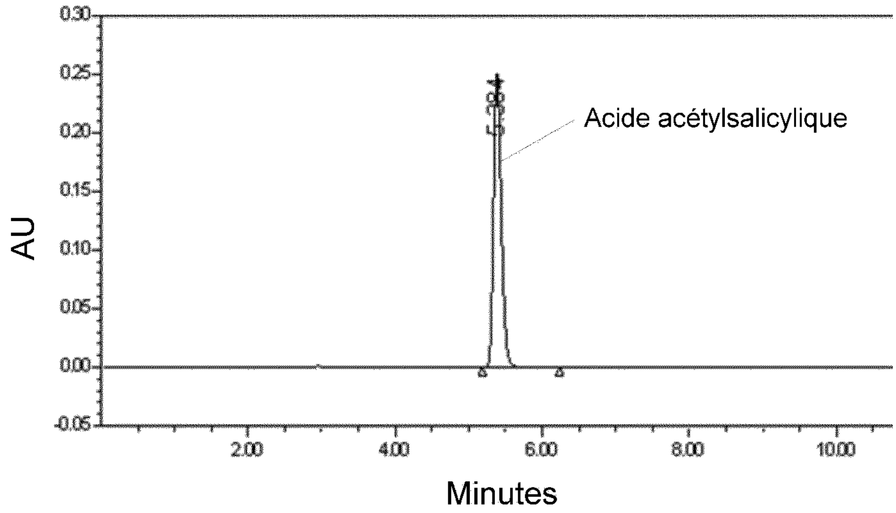
0.30
0.25
0.20
0.15
0.10
0.05
0.00
-0.05
AU
5.384
Acide acétylsalicylique
2.00
4.00
6.00
8.00
10.00
Minutes

FIG 1b

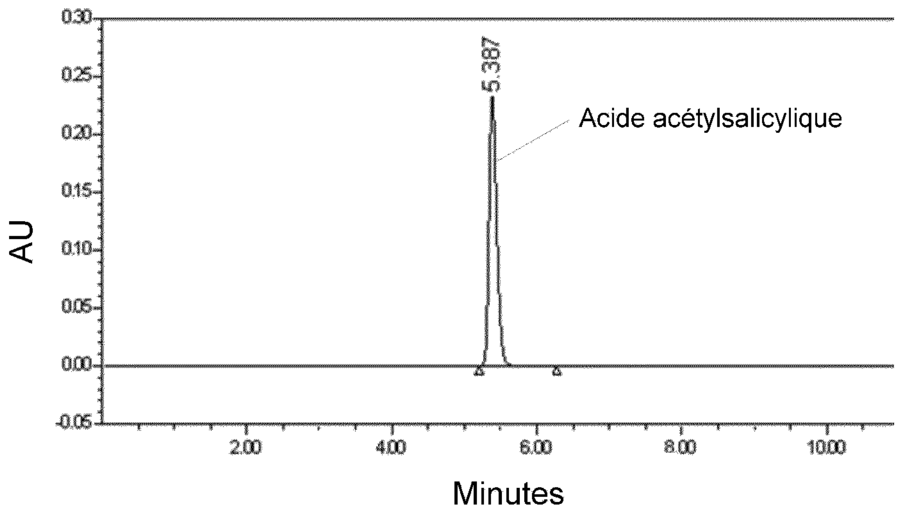
0.30
0.25
0.20
0.15
0.10
0.05
0.00
-0.05
AU
5.387
Acide acétylsalicylique
2.00
4.00
6.00
8.00
10.00
Minutes

FIG 2a

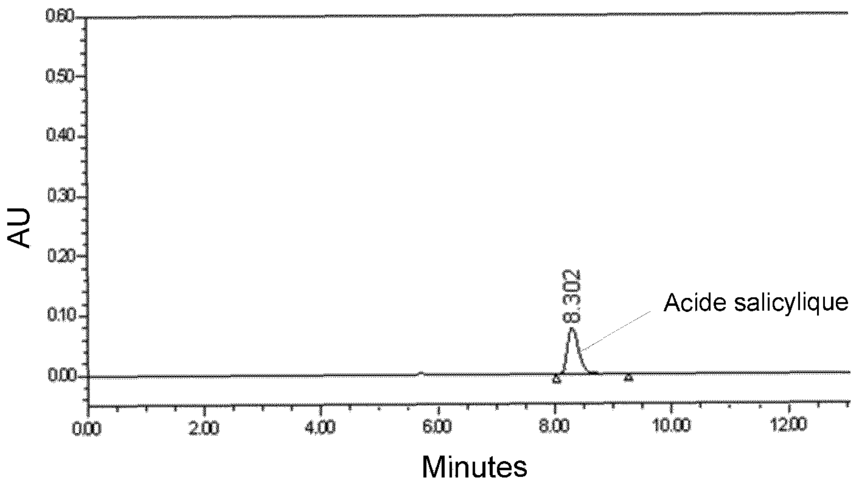


FIG 2b

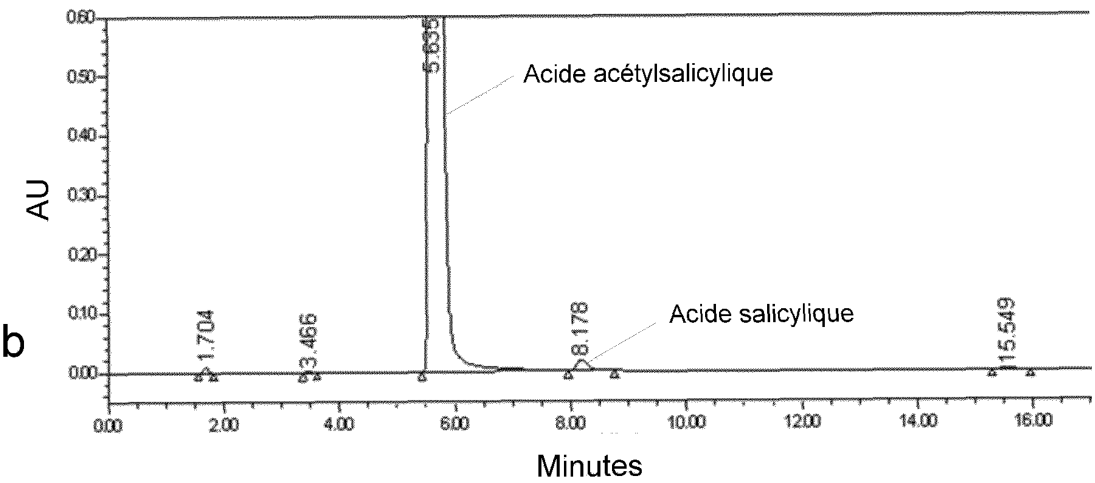


FIG 2c

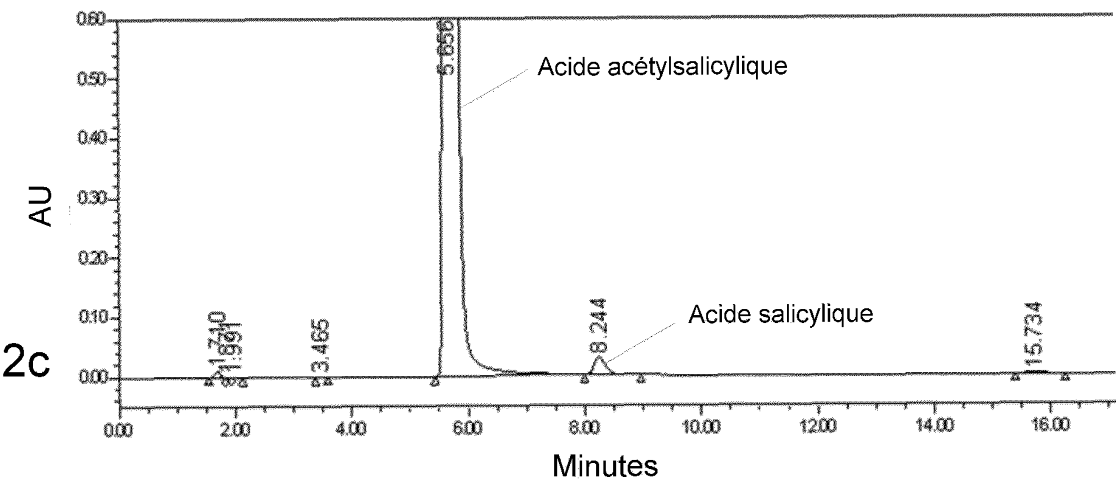

FIG 3

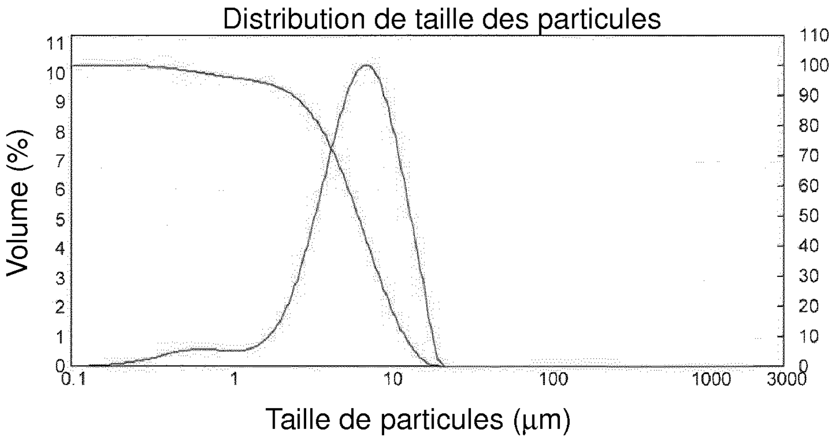


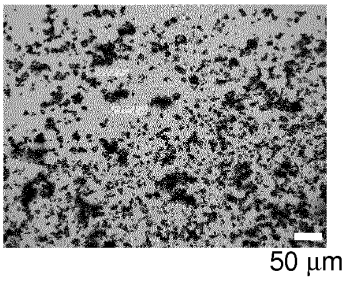


FIG 4

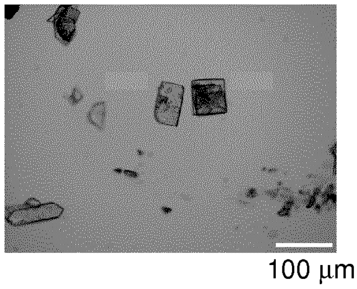


FIG 7

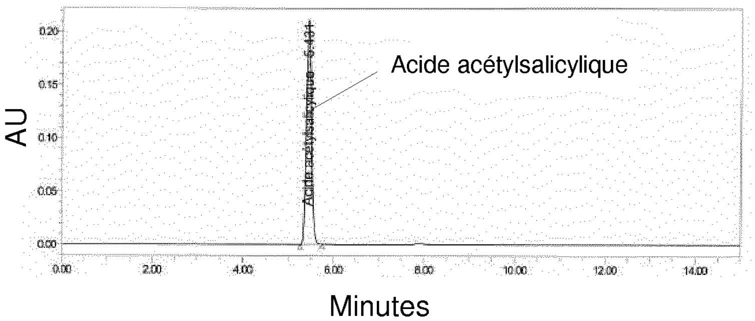


FIG 5

FIG 6

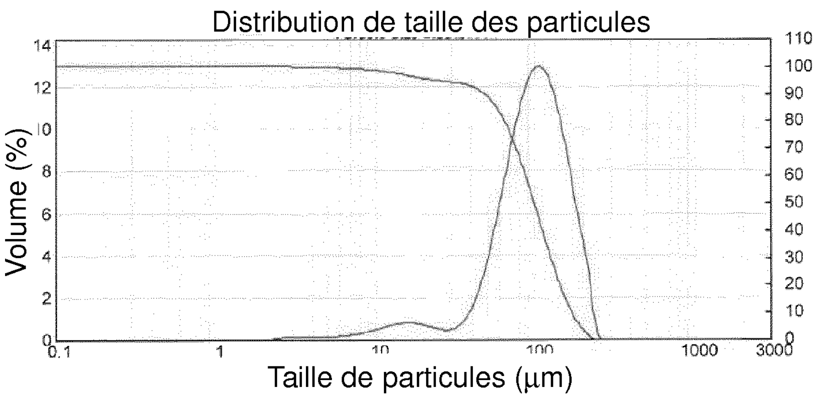

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2992641
- US 20020091108 A
- WO 2011039432 A
- FR 2973370